# EUROPEAN PATENT SPECIFICATION

(11) **EP 3 928 816 B1**
(45) Date of publication and mention of the grant of the patent: **04.03.2026**
(21) Application number: 20766414.5
(22) Date of filing: 06.03.2020
(51) Int. Cl.: A61M 5/24, A61M 5/315

(54) **INJECTION DEVICE**
INJEKTIONSVORRICHTUNG
DISPOSITIF D'INJECTION

(30) Priority: 18.02.2019 RU 2019104399
(43) Date of publication of application: 29.12.2021
(73) Proprietor: Limited Liability Company "Next Bio", Saint-Petersburg, 196608 (RU)
(72) Inventor: RODIONOV, Petr Petrovich, Leningradskaya obl., 188652 (RU); KAZEENKOV, Roman Sergeevich, Samarskaya obl., 455027 (RU); TARASENKO, Fedor Dmitrievich, Saint-Petersburg, 197022 (RU); ZHMAYLO, Michail Alexandrovich, Saint-Petersburg, 194358 (RU); HAFIZOV, Ruslan Ildarovich, Surgut Hanty-Mansiyskiy AO, 628416 (RU)
(74) Representative: Synergy IP Group AG
(86) International application number: PCT/RU2020/000120
(87) International publication number: WO 2020/180212

(56) References cited:
- EP-A2- 1 570 876
- EP-A2- 2 983 765
- WO-A1-2010/139640
- WO-A1-2013/119132
- WO-A1-2016/001306
- RU-C2- 2 551 933
- RU-C2- 2 671 419
- US-A- 5 383 865
- US-A1- 2014 088 515
- US-A1- 2014 088 515

## Description

### FIELD OF THE INVENTION

The invention relates to medical devices, in particular, to a device for injecting drugs into an organism, and can be implemented in the form of a syringe pen for self-administration of drugs.

### BACKGROUND OF THE INVENTION

Various designs of injection devices are known, in particular, devices made as syringe pens, which are most suitable for patients. Syringe pens are cylindrical injection devices convenient in shape for wearing and self-administration by patients. Such devices can comprise a needle, a cartridge with a drug, e.g. insulin or an analogue thereof, a hormone, an antihistamine or another drug, a rod with a drive, a dose-setting mechanism, and a push button.

Injection devices, including the devices made as syringe pens, should meet numerous requirements to be able to satisfy the needs of a patient. Said devices must be structurally durable yet at the same time convenient both in terms of use when manipulating device parts and in terms of the user's understanding of the operating principles of the device. Many diabetic or allergic patients may be physically infirm and may also have visual impairments, which can make it difficult to use a injection device. If the delivery device is a disposable device without the possibility of inserting another drug cartridge instead of a used and empty one, and hence is not reusable, the injection device should be cheap to manufacture and convenient in terms of disposal, preferably suitable for recycling.

In order to ensure user safety, such devices should be configured for automatically limiting the set dose in the event that a dose remaining in the cartridge is smaller than the patient requires. In this case, the dose of the drug required by the patient exceeds the dose remaining in the cartridge. Without such functionality in the device, a situation may arise wherein the dose of the drug required by the patient is not available in the cartridge, however, the device allows setting the dose-setting mechanism to an amount of the drug in excess of the amount of the drug remaining in the cartridge. In this situation, the patient may have a false sense of injecting the required amount of the drug which can lead to undesirable consequences, e.g., for patients with diabetes, acute allergic reactions or in need of emergency medical care. Therefore, injection devices need to be provided with a mechanism for preventing the dose of the drug from being set to an amount higher than the amount available in the drug cartridge.

One of the variations of a last dose limiter is implemented by providing mechanical summation of the administered doses and blocking the device upon reaching a certain number of administered doses. This approach can be implemented, in particular, by converting the rotational motion of the dose-setting mechanism into translational motion of the limiting (stopping) member. **In** this case, there should be no translational motion of the limiting member when the dose is being administered. **In** other words, the limiting member must carry out translational motion when the dose is being set and must remain stationary when the dose is being administered. Thus, the limiting member mechanically summates the doses set and administered by the user. The sum of the doses is expressed in the degree of advancement of the limiting member. When the limiting member reaches a set end position, it stops the dose-setting mechanism in the direction of increasing the set dose.

**In** particular, the Russian Federation patent No. 2254878 (filed on June 27, 2005, conventional priority date: September 16, 1999) discloses a injection device comprising a housing holding a cartridge, an injection button for releasing a set dose, a piston rod for moving the piston inside the cartridge, and a limiting mechanism preventing setting of a dose exceeding the amount of liquid remaining in the cartridge, wherein the limiting mechanism comprises a dose-setting member connected with the injection button, and a drive member connected to the piston rod, the dose-setting member and the drive member being movable with respect to each other when the dose-setting member rotates during setting the dose, wherein the injection button can be displaced aside, and the dose-setting member and the drive member are configured for joint motion when the set dose is being released by moving the injection button in the opposite direction, wherein the drive member is provided with a track having an end wall defining a length depending on the total amount of the drug in the cartridge, wherein said track is engaged with a tracking member connected to the dose-setting member for tracking the rotation of the dose-setting member, the tracking member and the dose-setting member being configured for joint rotation with respect to the drive member when setting the dose, and the tracking member and the dose-setting member being configured for joint rotation with the drive member when the dose is being released. It can be seen from the description that the prior art device implements the principle wherein the rotational motion of the dose-setting member causes a rotational-translational motion of the limiting member (nut) along the thread on the drive member.

In the prior art device, the drive member is provided with a track having an end wall defining a length depending on the total amount of the drug in the cartridge, said track is engaged with a tracking (limiting) member connected to the dose-setting member for tracking the rotation of the dose-setting member, the tracking member and the dose-setting member being configured for joint rotation with respect to the drive member when setting the dose, and the tracking member and the dose-setting member being configured for joint rotation with the drive member when the dose is being released. The drive member is cylindrical, and the track has a helical shape and is engaged with a cam member connected to the dose-setting member having the form of a cylinder that is coaxial with the drive member.

In injection devices with cylindrical members and a similar kinematic scheme, the drive member performs rather complex functions (rod movement, engagement with the dose-setting mechanism, providing exclusively translational motion of the drive members when setting the dose) and therefore has a complex shape. The addition of a new element in the form of a track (thread) to such a complex shape would lead to the design becoming complex and unreliable, and would further contribute to a complex manufacturing process and increase the rejection rate in manufacturing of such a part. In particular, in plastics manufacturing, the complex shape of the drive part can lead to the development and use of a complex mold, which, in turn, can affect the accuracy of production, increase the risk of defects and increase manufacturing costs in general.

Further, a pen-shaped injection device disclosed in Russian Federation patent No. 2519966 (filed on June 20, 2014, conventional priority date: June 1, 2009) is known in the art, the device is provided with a dose-setting mechanism, wherein the rotational motion of the dose-setting member also causes a rotational-translational motion of a limiting member (nut) along the thread on the drive element. The thread on the drive member has a variable pitch. The disadvantages of this prior art device also include the complexities in manufacturing and configuring parts of the mechanism, and consequently, the labor intensity and increased manufacturing costs for the device.

A prior art injection device in the form of a syringe pen is disclosed in Russian Federation patent No. 2653780 (prototype), in particular the device of Fig 2B (published on May 14, 2018, conventional priority date: March 16, 2011), wherein the rotational motion of the dose-setting member causes translational motion of the limiting member in the form of a half nut slider along the longitudinal guide due to an external thread formed on the slider and an internal thread formed on the dose-setting member.

Features of analogous prior art solutions and the prototype common with the present device are: the housing, the cylindrical dose-setting mechanism, the cylindrical driving portion, the rod, the cylindrical dose-setting mechanism being mounted inside the housing, the cylindrical driving portion being mounted inside the cylindrical dose-setting mechanism coaxially therewith, wherein the cylindrical dose-setting mechanism is rotatably mounted with respect to the cylindrical driving portion when setting and adjusting the dose, and the cylindrical dose-setting mechanism and the cylindrical driving portion being configured for joint rotation and driving the rod in translational motion when a force is applied to the cylindrical dose-setting mechanism in the longitudinal direction when the dose is being injected.

**In** this syringe pen, the limiting member of the last dose limiter is a half nut with threads provided on the outer surface thereof. The threads match the corresponding threads provided on the inner surface of the master controller (dose setter). The last dose limiter engages one or two threads provided on the master controller. When setting the dose, when the master controller rotates with respect to the drive and, accordingly, with respect to the last dose limiter, the limiter slides distally for a distance corresponding to the dose being set due to the threaded connection with threads on the master controller. During injection, as the drive and the master controller are connected to each other and configured to rotate with respect to each other, the last dose limiter will be held in position with respect to the threads of the master controller. The last dose limiter moves distally when setting the dose until the distal edge thereof abuts against a spline on the inner surface of the master controller. **In** this position, the last dose limiter is prevented from further distal motion, and therefore, the master controller is prevented from further rotation to set an additional dose.

**In** such a device, the internal thread on the dose-setting element complicates the design and thus leads to a technologically complex manufacturing process. The dose-setting member has a complex shape due to the need to ensure sliding motion thereof in the threads of the housing, as well as mating with the injection button and with the device for engaging with the drive. Supplementing the shape of the dose-setting member with an internal thread would complicate the manufacturing process and increase the rejection rate in manufacturing of the part. **In** particular, in plastics manufacturing, the complex shape of the part would further necessitate a complex mold, which would increase complexity and labor intensity of manufacturing, cause an increase in rejection rate, and therefore increase the cost of the device.

The closest prior art of the present invention is an injection device (EP 2983765; A61M 5/24, A61M 5/315; 17 February 2016). It is to note that the injection device disclosed in EP 2983765 comprises: a housing; a rod designed to engage with a drug cartridge such that a drug is extracted therefrom when the rod is moved; a cylindrical dose-setting mechanism mounted in the housing; and a cylindrical driving portion mounted in the cylindrical dose-setting mechanism coaxially therewith, wherein the cylindrical dose-setting mechanism is designed to be rotated with respect to the cylindrical driving portion when setting a drug dose to be injected, wherein the cylindrical dose-setting mechanism and the cylindrical driving portion are designed to rotate conjointly so as to translationally move the rod when a force is applied to the cylindrical dose-setting mechanism in a longitudinal direction when injecting the set drug dose. Also, the cylindrical driving portion in the injection device of EP 2983765 is provided with a dose limiter comprising a threaded shaft and a nut, wherein the nut is arranged on the threaded shaft such that the nut is spirally moved on the threaded shaft when rotating the cylindrical dose-setting mechanism engaged with the nut for setting a drug dose to be injected. However, the nut in the injection device of EP 2983765 needs to be precisely positioned between the cylindrical dose-setting mechanism and the cylindrical driving portion when originally assembling the injection device, wherein the nut is required to be tightly constrained to the cylindrical dose-setting mechanism, the threaded interface between the nut and the cylindrical driving portion is required to be preliminary manufactured in a precise manner. Otherwise, the nut in the injection device of EP 2983765 may be blocked between the cylindrical dose-setting mechanism and the cylindrical driving portion, thereby preventing the dose limiter from functioning correctly. Thus, a technical problem to be solved by the present invention is preventing the nut from being blocked between the cylindrical dose-setting mechanism and the cylindrical driving portion. A further technical problem to be solved by the present invention is to develop an alternative to the injection device disclosed in EP 2983765 that would be provided with a dose limiter preventing a drug dose exceeding a pre-determined sum of doses from being set by a user of the injection device.

### SUMMARY OF THE INVENTION

The invention is defined by the subject-matter of independent claims 1 and 9. Further embodiments are defined in the dependent claims. To solve the technical problem, there is provided an injection device, comprising:
a housing,
a rod designed to engage with a drug cartridge such that a drug is extracted therefrom when the rod is moved,
a cylindrical dose-setting mechanism mounted in the housing and
a cylindrical driving portion mounted in the cylindrical dose-setting mechanism coaxially therewith,
wherein the cylindrical dose-setting mechanism is designed to be rotated with respect to the cylindrical driving portion when setting a drug dose to be injected,
wherein the cylindrical dose-setting mechanism and the cylindrical driving portion are designed to rotate conjointly so as to translationally move the rod when a force is applied to the cylindrical dose-setting mechanism in a longitudinal direction when injecting the set drug dose,
wherein the cylindrical driving portion is provided with a cavity having a dose limiter arranged therein longitudinally with respect to an axis of the cylindrical driving portion,
wherein the dose limiter comprises a threaded shaft provided with a gear wheel, and a nut,
wherein the threaded shaft is rotatably mounted parallel to the axis of the cylindrical driving portion,
wherein an inner surface of the cylindrical driving portion is provided with a longitudinal groove oriented along the axis of the cylindrical driving portion, and the nut is provided with a protrusion received in the longitudinal groove,
wherein the nut is arranged on the threaded shaft such that the nut is translationally moved on the threaded shaft when rotating the threaded shaft, while the protrusion is moved along the longitudinal groove when translationally moving the nut,
wherein an inner surface of the cylindrical dose-setting mechanism is designed to engage with the gear wheel such that the threaded shaft is rotated when rotating the cylindrical dose-setting mechanism with respect to the cylindrical driving portion.

To solve the technical problem, there is also provided an injection device, comprising:
a housing,
a rod designed to engage with a drug cartridge such that a drug is extracted therefrom when the rod is moved,
a cylindrical dose-setting mechanism mounted in the housing and
a cylindrical driving portion mounted in the cylindrical dose-setting mechanism coaxially therewith,
wherein the cylindrical dose-setting mechanism is designed to be rotated with respect to the cylindrical driving portion when setting a drug dose to be injected,
wherein the cylindrical dose-setting mechanism and the cylindrical driving portion are designed to rotate conjointly so as to translationally move the rod when a force is applied to the cylindrical dose-setting mechanism in a longitudinal direction when injecting the set drug dose,
wherein the cylindrical driving portion is provided with a cavity having a dose limiter arranged therein longitudinally with respect to an axis of the cylindrical driving portion,
wherein the dose limiter comprises a threaded shaft provided with a gear wheel, and a nut,
wherein the threaded shaft is rotatably mounted parallel to the axis of the cylindrical driving portion,
wherein an inner surface of the cylindrical driving portion is provided with a longitudinal protrusion oriented along the axis of the cylindrical driving portion, and the nut is provided with a recess having the longitudinal protrusion of the cylindrical driving portion matched therewith,
wherein the nut is arranged on the threaded shaft such that the nut is translationally moved on the threaded shaft along the longitudinal protrusion when rotating the threaded shaft,
wherein an inner surface of the cylindrical dose-setting mechanism is designed to engage with the gear wheel such that the threaded shaft is rotated when rotating the cylindrical dose-setting mechanism with respect to the cylindrical driving portion

A technical effect provided by the present invention is improved reliability of the dose limiter used in the injection device according to the present invention due to simplified manufacture of the injection device, simplified and increased structural stability of the injection device and simplified assembly of the injection device. The provided economic effect is the low manufacturing cost and the low cost of the device compared to the prior art solutions. A further technical effect provided by the present invention is simplified replaceability of the dose limiter due to simplified access to the threaded shaft with the gear wheel.

**In** the device according to the invention, the nut can comprise at least one protrusion on the outer surface thereof, and a groove is made on the inner side of the cylindrical driving portion, the groove being oriented along the axis of the cylindrical driving portion, wherein said protrusion is configured to be received in said groove. Furthermore, the nut comprises at least one recess on the outer surface, and at least one protrusion is made in the cavity, wherein the nut is mounted in such manner that the recess on the surface thereof is matched with the longitudinal protrusion made in the cavity, providing limited translational motion and limited rotation of the nut.

Further, the injection device according to the present invention may further comprise a push button enabling a force to be applied in a longitudinal direction to the cylindrical dose-setting mechanism when injecting the dose.

An inner side of the cylindrical dose-setting mechanism in the injection device according to the present invention may be further provided with grooves, and the grooves and the gear wheel of the cylindrical driving portion may form a clutch mechanism, and the grooves and the gear wheel may be designed to engage with each other when the push button is pressed, and the cylindrical dose-setting mechanism and the cylindrical driving portion may be designed to engage by means of the clutch mechanism providing joint rotation thereof and driving the rod in translational motion when the push button is pressed.

The cylindrical mechanism and the cylindrical driving portion in the injection device according to the present invention may be designed to disengage when setting or adjusting the dose, while the button is released.

The threaded shaft in the device according to the invention may be formed integrally with the gear wheel.

The cylindrical dose-setting mechanism and the cylindrical driving portion in the device according to the invention may be each formed integrally.

The injection device according to the invention may be formed as a syringe pen.

The injection device according to the invention may be disposable, i.e. disposable devices with a single placement of a cartridge with a drug into the device, as well as non-separable syringe pens or disposable assembly syringe pens.

### BRIEF DESCRIPTION OF THE DRAWINGS

The invention will now be described in more detail with reference to the accompanying drawings, wherein:

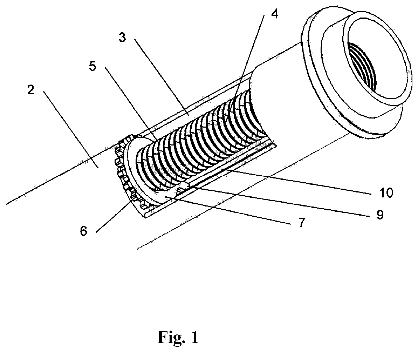

Fig. 1 schematically shows the last dose limiter mechanism without the cylindrical dose-setting mechanism.
Fig. 2 schematically shows the cylindrical dose-setting mechanism.

The figures show one of the potential embodiments of the injection device according to the present invention.

### DETAILED DESCRIPTION OF THE INVENTION

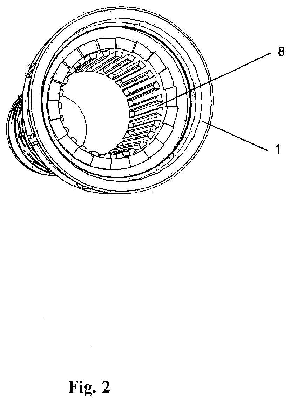

According to a non-limiting embodiment, the injection device comprises a housing (not shown in the drawings), a cylindrical dose-setting mechanism 1 (Fig. 2), a cylindrical driving portion 2, and a rod (not shown in the drawings) with a drug cartridge mounted in front of the rod (the cartridge usually containing a drug solution). The cylindrical dose-setting mechanism 1 is mounted inside the housing, and the cylindrical driving portion 2 is mounted inside the cylindrical dose-setting mechanism 1 coaxially therewith. Furthermore, the cylindrical dose-setting mechanism 1 is configured to rotate with respect to the cylindrical driving portion 2 when setting and adjusting a dose. The cylindrical dose-setting mechanism 1 and the cylindrical driving portion 2 are configured for joint rotation and driving the rod in translational motion when a force is applied to the cylindrical dose-setting mechanism 1 in a longitudinal direction when injecting the dose (when the rod is moved, and a required amount of the drug is extracted from the cartridge), wherein the cylindrical driving portion 2 is provided with a cavity 3 having a dose limiter arranged therein longitudinally with respect to an axis of the cylindrical driving portion 2, and the dose limiter comprises a shaft 4 provided with threads 5, a gear wheel 6, and a nut 7. The shaft 4 provided with threads 5 is rotatably mounted parallel to the axis of the cylindrical driving portion 2, and the gear wheel 6 is rigidly fastened to the shaft 4, and the nut 7 is mounted on the shaft 4 so as to be capable of translational motion and rotation. An inner surface of the cylindrical dose-setting mechanism 1 is provided with longitudinal grooves 8 (Fig. 2) designed to engage with the gear wheel 6. Thus, the cylindrical dose-setting mechanism 1 engages with the cylindrical driving portion 2.

The nut 7 is mounted so as to be capable of translational motion and rotation, wherein the nut 7 further comprises at least one protrusion 9 provided on an outer surface of the nut 7, and the protrusion 9 is received in a groove 10 provided on an inner surface of the cylindrical driving portion and is oriented along the axis of the cylindrical driving portion. Due to the placement of the protrusion 9 in the groove 10, the rotation of the nut 7 is limited by dimensions of the groove 10.

Both the engagement and the disengagement of the cylindrical dose-setting mechanism 1 and the cylindrical driving portion 2 are carried out by means of a clutch mechanism and a push button by using any method known in the art. The cylindrical mechanism 1 and the cylindrical driving portion 2 can be modified without departing from the scope of the present invention to provide interaction with the clutch mechanism and the push button. Such modifications are known in the art, in particular from the aforementioned analogues to the device according to the invention.

Parts of injection device or a syringe pen comprising the injection device of the present invention can be made of the following materials: polyethylene, polypropylene, polyoxymethylene (POM), ABS (acrylonitrile butadiene styrene), polystyrene or other rigid plastic or polymer materials or other materials, in particular, metals or alloys thereof.

The gear wheel 6 can be fixed on the shaft 4 during casting or press molding, thus forming a single piece comprising the shaft 4 with threads 5 and the gear wheel 6. The cylindrical dose-setting mechanism 1 and the cylindrical driving portion 2 having the cavity 3 formed therein can similarly be cast as single integral pieces.

During assembly, the nut 7 is mounted on the shaft 4 provided with the gear wheel 6. Then, the assembled dose-setting mechanism 1 is mounted inside the cylindrical driving portion 2.

The cylindrical driving portion 2 along with the clutch mechanism 1 and the push button (not shown in the drawings) are mounted inside the cylindrical dose-setting mechanism 1. All parts of the injection device are mounted inside the housing.

The injection device is operated as follows. The user sets a required dose of a drug to be administered by rotating the cylindrical dose-setting mechanism 1. The cylindrical dose-setting mechanism 1 rotates, while the cylindrical driving portion 2 remains stationary. After setting the required dose, the user applies a force, e.g. by pressing on the push button, leading to the engagement between the cylindrical dose-setting mechanism 1 and the cylindrical driving portion 2 by means of the clutch mechanism (not shown in the drawings). Further pressing of the push button by the user causes rotation of the cylindrical dose-setting mechanism 1 and rotation of the cylindrical driving portion 2 jointly therewith, and, consequently, leads to translational motion of the rod. The cylindrical dose-setting mechanism 1 thus moves to the initial position. The initial position can be considered as the position in which the described components of the device are in a rest position without the application of force by the user, or as the position in which the cylindrical dose-setting mechanism is not set to administer a dose of any volume (a dose of zero volume). During translational motion, the rod presses on the drug cartridge, e.g. by pushing the closing plug or the bottom of the drug cartridge, resulting in a predetermined amount of the drug flowing from the cartridge through the needle and out of the device, thus allowing the dose to be administered to the patient.

When the cylindrical dose-setting mechanism 1 rotates, the gear wheel 6 mounted on the shaft 4 also rotates along with the shaft 4 and engages with the grooves 8 made on the inner surface of the cylindrical mechanism 1. Therefore, the cylindrical dose-setting mechanism and the cylindrical driving portion engage with each other. When the shaft 4 rotates, the nut 7 moves translationally, while the protrusion 9 moves along the groove 10. When the end of the groove 10 is reached, further motion of the nut 7 is impossible, thus stopping the shaft 4 and the gear wheel 6 thereon. The rotation of the cylindrical dose-setting mechanism 1 ceases, thus stopping the mechanism. Therefore, the user is prevented from setting a dose exceeding the sum of doses corresponding to the length of the groove 10.

**In** addition to reliably limiting the last dose, the device comprises simple parts, both from the manufacturing standpoint and from the design standpoint.

The process of manufacturing the cylindrical driving portion 2 with the cavity 3 formed therein is carried out without forming threads on said portion. **In** the event that the cylindrical driving portion is made of, e.g. a polymer material, the rejection rate is minimal.

The shaft 4 with threads 5, the gear wheel 6, and the nut 7 disclosed in the present device are similarly reliable and easy to manufacture.

The cylindrical dose-setting mechanism 1 comprising longitudinal grooves 8 for engagement with the gear wheel 6 of the dose limiter provided on the inner surface thereof is similarly reliable and rather easy to manufacture and does not require forming an internal thread which could lead to a high rejection rate during manufacturing.

The device according to the invention has been tested for administration of various drugs to patients, e.g. for subcutaneous administration of a drug selected from the group consisting of insulins, insulin analogs, hormones, heparins, antihistamines, and derivatives and analogs thereof. **In** each case, reliable dose limitation was achieved in the event of the dose remaining in the cartridge being smaller than that required by the patient.

The disclosed embodiments are non-limiting, with the scope of protection of the present invention limited solely by the appended claims. It will be apparent to those skilled in the art that other embodiments are possible within the scope of the claims.

## Claims

1. An injection device, comprising:
a housing,
a rod designed to engage with a drug cartridge such that a drug is extracted therefrom when the rod is moved,
a cylindrical dose-setting mechanism (1) mounted in the housing and
a cylindrical driving portion (2) mounted in the cylindrical dose-setting mechanism (1) coaxially therewith,
wherein the cylindrical dose-setting mechanism (1) is designed to be rotated with respect to the cylindrical driving portion (2) when setting a drug dose to be injected,
wherein the cylindrical dose-setting mechanism (1) and the cylindrical driving portion (2) are designed to rotate conjointly so as to translationally move the rod when a force is applied to the cylindrical dose-setting mechanism (1) in a longitudinal direction when injecting the set drug dose,
**characterized in that**
the cylindrical driving portion (2) is provided with a cavity (3) having a dose limiter arranged therein longitudinally with respect to an axis of the cylindrical driving portion (2), and
the dose limiter comprises a threaded shaft (4) provided with a gear wheel (6), and a nut (7),
wherein the threaded shaft (4) is rotatably mounted parallel to the axis of the cylindrical driving portion (2),
wherein an inner surface of the cylindrical driving portion (2) is provided with a longitudinal groove (10) oriented along the axis of the cylindrical driving portion (2), and the nut (7) is provided with a protrusion (9) received in the longitudinal groove (10),
wherein the nut (7) is arranged on the threaded shaft (4) such that the nut (7) is translationally moved on the threaded shaft (4) when rotating the threaded shaft (4), while the protrusion (9) is moved along the longitudinal groove (10) when translationally moving the nut (7),
wherein an inner surface of the cylindrical dose-setting mechanism (1) is designed to engage with the gear wheel (6) such that the threaded shaft (4) is rotated when rotating the cylindrical dose-setting mechanism (1) with respect to the cylindrical driving portion (2).

2. The device according to claim 1, further comprising a push button enabling a force to be applied in the longitudinal direction to the cylindrical dose-setting mechanism (1) when injecting the dose.

3. The device according to claim 2, wherein
an inner side of the cylindrical dose-setting mechanism (1) is provided with grooves (8), and
the grooves (8) and the gear wheel (6) of the cylindrical driving portion (2) form a clutch mechanism, and
the grooves (8) and the gear wheel (6) are designed to engage with each other when the push button is pressed, and
the cylindrical dose-setting mechanism (1) and the cylindrical driving portion (2) are designed to engage by means of the clutch mechanism providing joint rotation thereof and driving the rod in translational motion when the push button is pressed.

4. The device according to any one of the claims 2-3, wherein the cylindrical dose-setting mechanism (1) and the cylindrical driving portion (2) are designed to disengage when setting or adjusting the dose, while the button is released.

5. The device according to any one of the claims 1-4, wherein the threaded shaft (4) is formed integrally with the gear wheel (6).

6. The device according to any one of the claims 1-5, wherein the cylindrical dose-setting mechanism (1) and the cylindrical driving portion (2) are each formed integrally.

7. The device according to any one of the claims 1-6, wherein the device is formed as a syringe pen.

8. The device according to any one of the claims 1-6, wherein the device is disposable.

9. An injection device, comprising:
a housing,
a rod designed to engage with a drug cartridge such that a drug is extracted therefrom when the rod is moved,
a cylindrical dose-setting mechanism (1) mounted in the housing and
a cylindrical driving portion (2) mounted in the cylindrical dose-setting mechanism (1) coaxially therewith,
wherein the cylindrical dose-setting mechanism (1) is designed to be rotated with respect to the cylindrical driving portion (2) when setting a drug dose to be injected,
wherein the cylindrical dose-setting mechanism (1) and the cylindrical driving portion (2) are designed to rotate conjointly so as to translationally move the rod when a force is applied to the cylindrical dose-setting mechanism (1) in a longitudinal direction when injecting the set drug dose,
**characterized in that**
the cylindrical driving portion (2) is provided with a cavity (3) having a dose limiter arranged therein longitudinally with respect to an axis of the cylindrical driving portion (2), and
the dose limiter comprises a threaded shaft (4) provided with a gear wheel (6), and a nut (7),
wherein the threaded shaft (4) is rotatably mounted parallel to the axis of the cylindrical driving portion (2),
wherein an inner surface of the cylindrical driving portion (2) is provided with a longitudinal protrusion oriented along the axis of the cylindrical driving portion (2), and the nut (7) is provided with a recess having the longitudinal protrusion of the cylindrical driving portion (2) matched therewith,
wherein the nut (7) is arranged on the threaded shaft (4) such that the nut (7) is translationally moved on the threaded shaft (4) along the longitudinal protrusion when rotating the threaded shaft (4),
wherein an inner surface of the cylindrical dose-setting mechanism (1) is designed to engage with the gear wheel (6) such that the threaded shaft (4) is rotated when rotating the cylindrical dose-setting mechanism (1) with respect to the cylindrical driving portion (2).

## Patentansprüche

1. Eine Injektionsvorrichtung, umfassend:
ein Gehäuse,
einen Stab, der dazu ausgestaltet ist, mit einer Arzneimittelpatrone in Eingriff zu stehen,
sodass ein Arzneimittel daraus extrahiert wird, wenn der Stab bewegt wird,
einen zylindrischen Dosiseinstellmechanismus (1), der im Gehäuse angebracht ist, und
einen zylindrischen Antriebsteil (2), der koaxial im zylindrischen Dosiseinstellmechanismus (1) angebracht ist,
wobei der zylindrische Dosiseinstellmechanismus (1) dazu ausgestaltet ist, relativ zum zylindrischen Antriebsteil (2) gedreht zu werden, wenn eine zu injizierende Arzneimitteldosis eingestellt wird,
wobei der zylindrische Dosiseinstellmechanismus (1) und der zylindrische Antriebsteil (2) dazu ausgestaltet sind, gemeinsam zu rotieren, um den Stab in eine translatorische Bewegung zu versetzen, wenn beim Injizieren der eingestellten Arzneimitteldosis eine Kraft in Längs-richtung auf den zylindrischen Dosiseinstellmechanismus (1) ausgeübt wird,
**dadurch gekennzeichnet, dass**
der zylindrische Antriebsteil (2) mit einer Aussparung (3) versehen ist, in der ein Dosisbegrenzer längs zu einer Achse des zylindrischen Antriebsteils (2) angeordnet ist, und der Dosisbegrenzer eine Gewindewelle (4) mit einem Zahnrad (6) sowie eine Mutter (7) umfasst,
wobei die Gewindewelle (4) drehbar parallel zur Achse des zylindrischen Antriebsteils (2) angebracht ist,
wobei eine innere Oberfläche des zylindrischen Antriebsteils (2) mit einer Längsnut (10) versehen ist, die sich entlang der Achse des zylindrischen Antriebsteils (2) erstreckt, und die Mutter (7) mit einem Vorsprung (9) versehen ist, der in der Längsnut (10) aufgenommen ist,
wobei die Mutter (7) auf der Gewindewelle (4) derart angeordnet ist, dass die Mutter (7) translatorisch auf der Gewindewelle (4) bewegt wird, wenn die Gewindewelle (4) gedreht wird, während sich der Vorsprung (9) beim translatorischen Bewegen der Mutter (7) entlang der Längsnut (10) bewegt,
wobei eine innere Oberfläche des zylindrischen Dosiseinstellmechanismus (1) dazu ausgestaltet ist, mit dem Zahnrad (6) in Eingriff zu treten, sodass die Gewindewelle (4) gedreht wird, wenn der zylindrische Dosiseinstellmechanismus (1) relativ zum zylindrischen Antriebsteil (2) gedreht wird.

2. Vorrichtung nach Anspruch 1, ferner umfassend eine Drucktaste, die es ermöglicht, beim Injizieren der Dosis eine Kraft in Längsrichtung auf den zylindrischen Dosiseinstellmechanismus (1) auszuüben.

3. Vorrichtung nach Anspruch 2, wobei
eine Innenseite des zylindrischen Dosiseinstellmechanismus (1) mit Nuten (8) versehen ist, und die Nuten (8) und das Zahnrad (6) des zylindrischen Antriebsteils (2) einen Kupplungsmechanismus bilden, und
die Nuten (8) und das Zahnrad (6) dazu ausgestaltet sind, miteinander in Eingriff zu treten, wenn die Drucktaste gedrückt wird, und
der zylindrische Dosiseinstellmechanismus (1) und der zylindrische Antriebsteil (2) dazu ausgestaltet sind, mittels des Kupplungsmechanismus derart miteinander in Eingriff zu treten, dass sie gemeinsam rotieren und den Stab beim Drücken der Drucktaste translatorisch antreiben.

4. Vorrichtung nach einem der Ansprüche 2-3, wobei der zylindrische Dosiseinstellmechanismus (1) und der zylindrische Antriebsteil (2) dazu ausgestaltet sind, beim Einstellen oder Anpassen der Dosis getrennt zu werden, während die Drucktaste losgelassen ist.

5. Vorrichtung nach einem der Ansprüche 1-4, wobei die Gewindewelle (4) in einem Stück mit dem Zahnrad (6) ausgebildet ist.

6. Vorrichtung nach einem der Ansprüche 1-5, wobei sowohl der zylindrische Dosiseinstellmechanismus (1) als auch der zylindrische Antriebsteil (2) jeweils in einem Stück ausgebildet sind.

7. Vorrichtung nach einem der Ansprüche 1-6, wobei die Vorrichtung als Injektionspen ausgebildet ist.

8. Vorrichtung nach einem der Ansprüche 1-6, wobei die Vorrichtung eine Einwegvorrichtung ist.

9. Eine Injektionsvorrichtung, umfassend:
ein Gehäuse,
einen Stab, der dazu ausgestaltet ist, mit einer Arzneimittelpatrone in Eingriff zu stehen, sodass ein Arzneimittel daraus extrahiert wird, wenn der Stab bewegt wird,
einen zylindrischen Dosiseinstellmechanismus (1), der im Gehäuse angebracht ist, und
einen zylindrischen Antriebsteil (2), der koaxial im zylindrischen Dosiseinstellmechanismus (1) angebracht ist,
wobei der zylindrische Dosiseinstellmechanismus (1) dazu ausgestaltet ist, relativ zum zylindrischen Antriebsteil (2) gedreht zu werden, wenn eine zu injizierende Arzneimitteldosis eingestellt wird,
wobei der zylindrische Dosiseinstellmechanismus (1) und der zylindrische Antriebsteil (2) dazu ausgestaltet sind, gemeinsam zu rotieren, um den Stab in eine translatorische Bewegung zu versetzen, wenn beim Injizieren der eingestellten Arzneimitteldosis eine Kraft in Längs-richtung auf den zylindrischen Dosiseinstellmechanismus (1) ausgeübt wird,
**dadurch gekennzeichnet, dass**
der zylindrische Antriebsteil (2) mit einer Aussparung (3) versehen ist, in der ein Dosisbegrenzer längs zu einer Achse des zylindrischen Antriebsteils (2) angeordnet ist, und der Dosisbegrenzer eine Gewindewelle (4) mit einem Zahnrad (6) sowie eine Mutter (7) umfasst,
wobei die Gewindewelle (4) drehbar parallel zur Achse des zylindrischen Antriebsteils (2) angebracht ist,
wobei eine innere Oberfläche des zylindrischen Antriebsteils (2) mit einem längs verlaufenden Vorsprung versehen ist, der entlang der Achse des zylindrischen Antriebsteils (2) orientiert ist, und die Mutter (7) eine Vertiefung aufweist, die mit dem längs verlaufenden Vorsprung des zylindrischen Antriebsteils (2) korrespondiert, wobei die Mutter (7) auf der Gewindewelle (4) derart angeordnet ist, dass die Mutter (7) translatorisch auf der Gewindewelle (4) entlang des längs verlaufenden Vorsprungs bewegt wird, wenn die Gewindewelle (4) gedreht wird,
wobei eine innere Oberfläche des zylindrischen Dosiseinstellmechanismus (1) dazu ausgestaltet ist, mit dem Zahnrad (6) in Eingriff zu treten, sodass die Gewindewelle (4) gedreht wird, wenn der zylindrische Dosiseinstellmechanismus (1) relativ zum zylindrischen Antriebsteil (2) gedreht wird.

## Revendications

1. Dispositif d'injection comprenant :
un logement,
une tige conçue pour s'engager avec une cartouche de médicament de sorte qu'un médicament soit extrait de celle-ci lorsque la tige est déplacée,
un mécanisme de dosage cylindrique (1) monté dans le logement et
une partie d'entraînement cylindrique (2) montée dans le mécanisme de dosage cylindrique (1) coaxialement à celui-ci,
le mécanisme de dosage cylindrique (1) étant conçu pour tourner par rapport à la partie d'entraînement cylindrique (2) lors du dosage d'un médicament à injecter,
le mécanisme de dosage cylindrique (1) et la partie d'entraînement cylindrique (2) étant conçus pour tourner conjointement afin de déplacer la tige en translation lorsqu'une force est appliquée sur le mécanisme de dosage cylindrique (1) dans une direction longitudinale lors de l'injection du médicament dosé,
**caractérisé en ce que**
la partie cylindrique d'entraînement (2) est munie d'une cavité (3) comportant un limiteur de dose disposé à l'intérieur longitudinalement à un axe de la partie d'entraînement cylindrique (2), et
le limiteur de dose comprend un arbre fileté (4) muni d'une roue dentée (6) et d'un écrou (7),
l'arbre fileté (4) étant monté de manière rotative parallèlement à l'axe de la partie d'entraînement cylindrique (2),
une surface intérieure de la partie d'entraînement cylindrique (2) étant munie d'une rainure longitudinale (10) orientée suivant l'axe de la partie d'entraînement cylindrique (2), et l'écrou (7) étant muni d'une saillie (9) reçue dans la rainure longitudinale (10),
l'écrou (7) étant disposé sur l'arbre fileté (4) de telle sorte que l'écrou (7) soit déplacé en translation sur l'arbre fileté (4) lors de la rotation de l'arbre fileté (4), tandis que la saillie (9) est déplacée le long de la rainure longitudinale (10) lors du déplacement de l'écrou (7) en translation,
une surface intérieure du mécanisme de dosage cylindrique (1) étant conçue pour s'engager avec la roue dentée (6) de sorte que l'arbre fileté (4) tourne lors de la rotation du mécanisme de dosage cylindrique (1) par rapport à la partie d'entraînement cylindrique (2).

2. Dispositif selon la revendication 1, comprenant en outre un bouton-poussoir permettant d'appliquer une force dans la direction longitudinale sur le mécanisme de dosage cylindrique (1) lors de l'injection de la dose.

3. Dispositif selon la revendication 2,
un côté intérieur du mécanisme de dosage cylindrique (1) étant muni de rainures (8), et
les rainures (8) et la roue dentée (6) de la partie d'entraînement cylindrique (2) formant un mécanisme d'embrayage, et
les rainures (8) et la roue dentée (6) étant conçues pour s'engager l'une avec l'autre lorsque le bouton-poussoir est enfoncé, et
le mécanisme de dosage cylindrique (1) et la partie d'entraînement cylindrique (2) étant conçus pour s'engager au moyen du mécanisme d'embrayage ce qui assure une rotation conjointe de ceux-ci et entraîne la tige suivant un mouvement de translation lorsque le bouton-poussoir est enfoncé.

4. Dispositif selon l'une quelconque des revendications 2 et 3, le mécanisme de dosage cylindrique (1) et la partie d'entraînement cylindrique (2) étant conçus pour se désengager lors du dosage ou de l'ajustement de la dose, tandis que le bouton est relâché.

5. Dispositif selon l'une quelconque des revendications 1 à 4, l'arbre fileté (4) étant formé de manière solidaire avec la roue dentée (6).

6. Dispositif selon l'une quelconque des revendications 1 à 5, le mécanisme de dosage cylindrique (1) et la partie d'entraînement cylindrique (2) étant chacun formés d'une seule pièce.

7. Dispositif selon l'une quelconque des revendications 1 à 6, le dispositif étant réalisé sous la forme d'un stylo seringue.

8. Dispositif selon l'une quelconque des revendications 1 à 6, le dispositif étant jetable.

9. Dispositif d'injection comprenant :
un logement,
une tige conçue pour s'engager avec une cartouche de médicament de sorte qu'un médicament soit extrait de celle-ci lorsque la tige est déplacée,
un mécanisme de dosage cylindrique (1) monté dans le logement et
une partie d'entraînement cylindrique (2) montée dans le mécanisme de dosage cylindrique (1) coaxialement à celui-ci,
le mécanisme de dosage cylindrique (1) étant conçu pour tourner par rapport à la partie d'entraînement cylindrique (2) lors du dosage d'un médicament à injecter,
le mécanisme de dosage cylindrique (1) et la partie d'entraînement cylindrique (2) étant conçus pour tourner conjointement afin de déplacer la tige en translation lorsqu'une force est appliquée sur le mécanisme de dosage cylindrique (1) dans une direction longitudinale lors de l'injection du médicament dosé,
**caractérisé en ce que**
la partie d'entraînement cylindrique (2) est munie d'une cavité (3) comportant un limiteur de dose disposé longitudinalement à un axe de la partie d'entraînement cylindrique (2), et
le limiteur de dose comprend un arbre fileté (4) muni d'une roue dentée (6) et d'un écrou (7),
l'arbre fileté (4) étant monté de manière rotative parallèlement à l'axe de la partie d'entraînement cylindrique (2),
une surface intérieure de la partie d'entraînement cylindrique (2) étant munie d'une saillie longitudinale orientée suivant l'axe de la partie d'entraînement cylindrique (2), et l'écrou (7) étant muni d'un logement comportant la saillie longitudinale de la partie d'entraînement cylindrique (2) correspondant à celle-ci, l'écrou (7) étant disposé sur l'arbre fileté (4) de telle sorte que l'écrou (7) soit déplacé en translation sur l'arbre fileté (4) le long de la saillie longitudinale lors de la rotation de l'arbre fileté (4),
une surface intérieure du mécanisme de dosage cylindrique (1) étant conçue pour s'engager avec la roue dentée (6) de sorte que l'arbre fileté (4) tourne lors de la rotation du mécanisme de dosage cylindrique (1) par rapport à la partie d'entraînement cylindrique (2).
